(19) Europäisches Patentamt
European Patent Office
Office européen des brevets

(11) Veröffentlichungsnummer: **0 161 617**
**A2**

# (12) EUROPÄISCHE PATENTANMELDUNG

(21) Anmeldenummer: **85105540.0**

(22) Anmeldetag: **07.05.85**

(51) Int. Cl.⁴: **C 07 D 277/46**
// C07D501/20

(30) Priorität: **17.05.84 DE 3418376**

(43) Veröffentlichungstag der Anmeldung: **21.11.85**
**Patentblatt 85/47**

(84) Benannte Vertragsstaaten: **AT BE CH DE FR GB IT LI NL SE**

(71) Anmelder: **BAYER AG, Konzernverwaltung RP**
**Patentabteilung, D-5090 Leverkusen 1 Bayerwerk (DE)**

(72) Erfinder: **Boberg, Michael, Dr., Untere Bergerheide 47, D-5600 Wuppertal 1 (DE)**
Erfinder: **Naab, Paul, Dr. z.Zt Cutter Group, P.O. Box 8817 2200 Powell Street, Emeryville California 94662 (US)**
Erfinder: **Samaan, Samir, Dr., Claudiusweg 9, D-5600 Wuppertal 1 (DE)**

## (54) Zwischenprodukte sowie Verfahren zur Herstellung von Zwischenproducten für die Synthese von Cephalosporinen.

(57) Die Erfindung betrifft neue 2-(2-Acylamido-thiazol-4-yl)-2-butensäurealkylester der Formeln

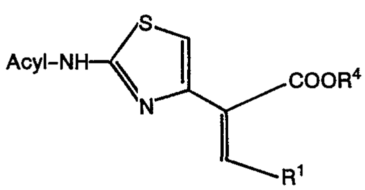

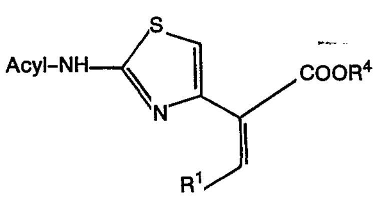

in denen Acyl und $R^1$ die in der Beschreibung angegebene Bedeutung haben sowie Verfahren zur Herstellung dieser Verbindungen und ihre Verwendung zur Herstellung von Cephalosporinen.

BAYER AKTIENGESELLSCHAFT 5090 Leverkusen, Bayerwerk

Konzernverwaltung RP
Patentabteilung Ad/Ke-c

# Zwischenprodukte sowie Verfahren zur Herstellung von Zwischenprodukten für die Synthese von Cephalosporinen

Die Erfindung betrifft neue 2-(2-Acylamido-thiazol-4-yl)-2-butensäurealkylester, ein Verfahren zu ihrer Herstellung sowie ihre Verwendung zur Herstellung von Cephalosporinen.

Cephalosporine der allgemeinen Formel I sind in der europäischen Patentanmeldung 49448 genannt. Diese Verbindungen haben eine breite antibakterielle Wirkung sowohl gegen gramnegative wie auch gegen grampositive Bakterien.

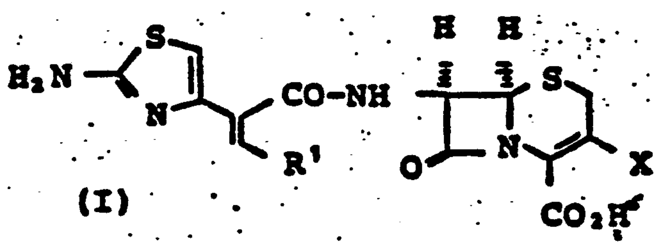

(I)

$R^1$ = Alkyl, Aryl

Nach dem in dieser Patentanmeldung genannten Verfahren werden die Verbindungen der Formel I nach dem in dem folgenden Formelschema aufgeführten Verfahren hergestellt:

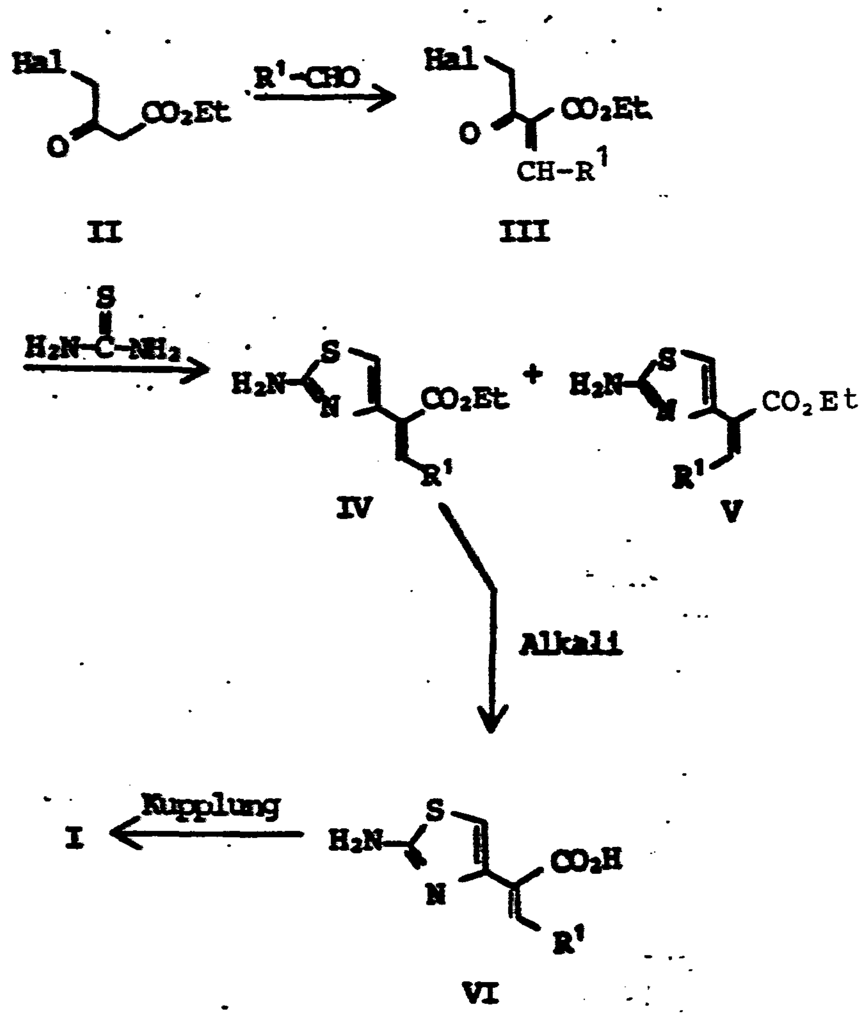


II, III, IV, V, VI, I

Dieses Verfahren führt jedoch für Verbindungen der Formel I mit $R^1$ = Alkyl nur zu unbefriedigenden Ausbeuten. So erhält man - z.B. für den Fall $R^1$ = Isopropyl - bei der Umsetzung von III mit Thioharnstoff durch Dekonjugation der Doppelbindung und durch Michaeladdition neben der Z-konfigurierten Verbindung IV und der E-konfigurierten Verbindung V die Produkte der Formeln VII und VIII zum bei weitem größten Anteil.

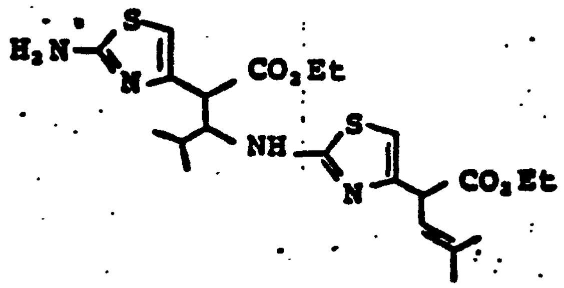

VIII

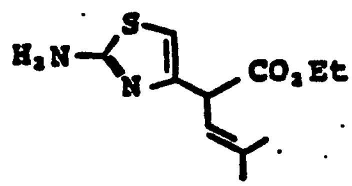

VII

Weiterhin wird in der europäischen Patentanmeldung 81 674 ein Verfahren zur Herstellung von Verbindungen der Formeln XIII und XIV beschrieben, welches für $R^1$ = Alkyl und $R^1$ = Aryl anwendbar ist und nach dem folgenden Formelschema abläuft:

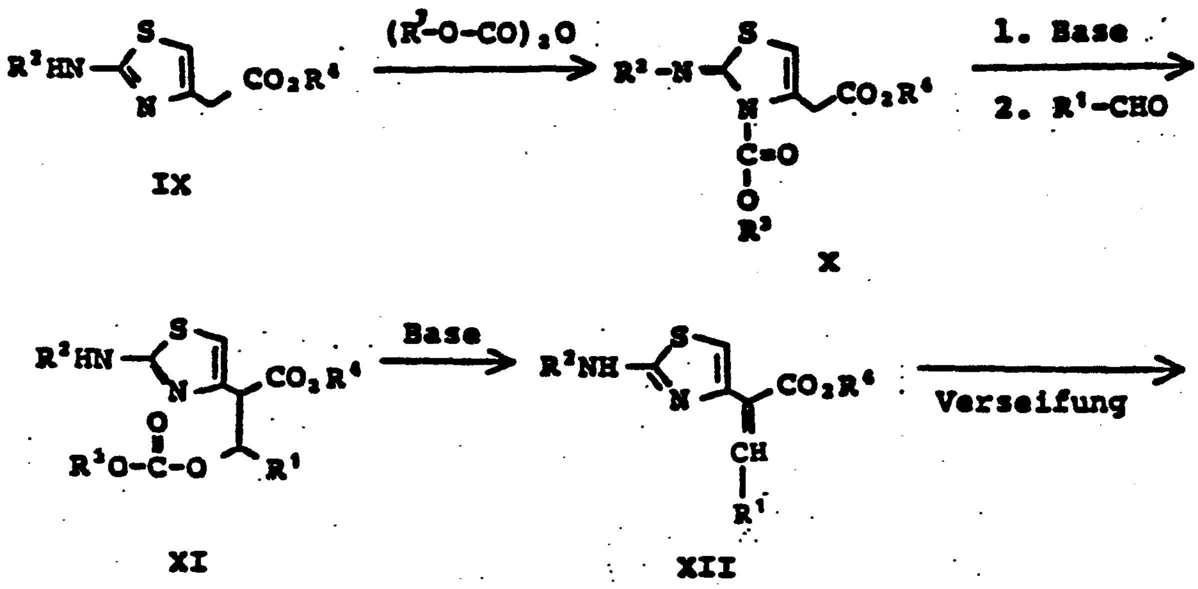


IX X XI XII

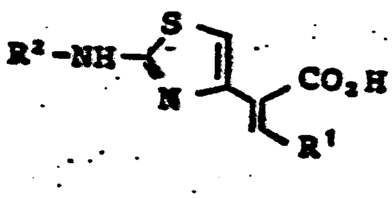

XIII

+

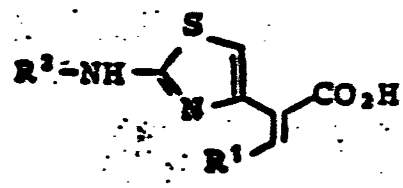

XIV

Aus XIII läßt sich zwar durch Abspaltung der Schutzgruppe $R^2$ prinzipiell die angestrebte Verbindung VI herstellen, jedoch benötigt dieses Verfahren zur Herstellung von VI infolge der Einführung und späteren Abspaltung der zwei Aminschutzgruppen $R^2$ und $COOR^3$ zusätzlich zur gesondert durchzuführenden Spaltung des Esters $CO_2R^4$ zur Carbonsäure mehr Synthesestufen als das vorangehend beschriebene Verfahren.

Es wurde nun ein Verfahren zur Herstellung der bevorzugten Z-konfigurierten Verbindung VI, in der $R^1$ = Alkyl, Alkenyl, Cycloalkyl oder Cycloalkenyl ist, gefunden, das über neue Zwischenprodukte der Formeln XVI und/oder XVII in nur wenigen Synthesestufen reinere Produkte liefert.

Überraschenderweise zeigte sich, daß bei der Umsetzung der 2-Halogenacetylacrylsäureester III statt mit Thioharnstoff mit einem Acylthioharnstoff XV, bevorzugt Acetylthioharnstoff, die Verbindungen XVI und XVII in hoher Reinheit entstehen und sehr einfach zu isolieren sind.

Zur Umsetzung geeignete Acylthioharnstoffe haben die Formel XV

$$\text{Acyl-HN-}\overset{S}{\overset{\|}{C}}\text{-NH}_2 \qquad \text{(XV)}$$

worin Acyl für Alkylcarbonyl, bevorzugt $C_1$-$C_6$ Alkylcarbonyl, worin Alkyl verzweigt oder unverzweigt und auch cyclisch sein kann und durch Halogen oder Aryl substituiert sein kann, wenn durch Aryl, dann bevorzugt Phenyl, Arylcarbonyl, bevorzugt Phenylcarbonyl oder durch Niedrigalkoxyl, Halogen oder Nitro substituiertes Phenylcarbonyl steht.

Die Herstellung der Zwischenprodukte XVI und XVII verläuft nach folgendem Schema:

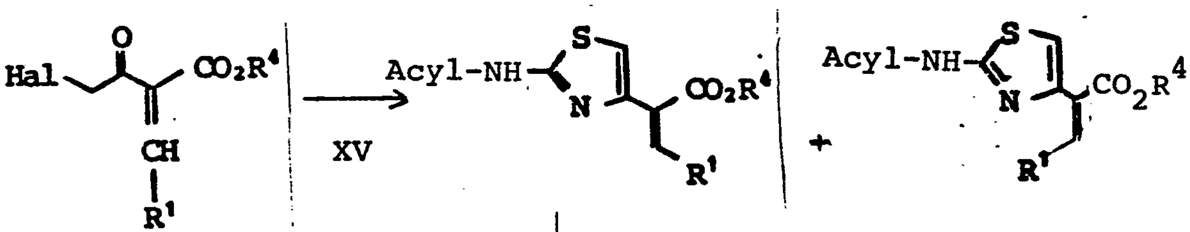


III XVI XVII

Hierin bedeuten

Acyl die bei XV genannten Reste,

$R^1$ einen gegebenenfalls substituierten verzweigten oder unverzweigten Alkyl-, Alkenyl-, Cycloalkyl- oder Cycloalkenyl-Rest und

$R^4$ einen gegebenenfalls substituierten, verzweigten oder unverzweigten $C_1$-$C_6$-Alkyl- oder Cycloalkylrest.

Gegenstand der Erfindung sind demnach Verbindungen der Formeln XVI und XVII

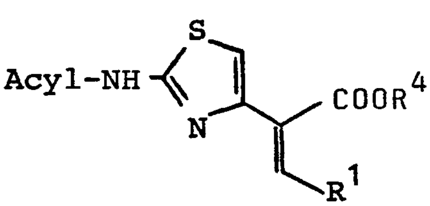

XVI

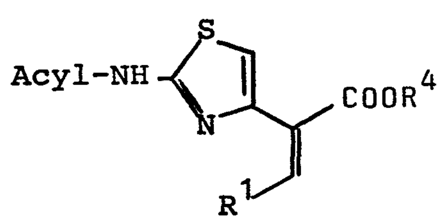

XVII

in denen

Acyl die bei XV genannten Reste,

$R^1$ einen gegebenenfalls substituierten, verzweigten oder unverzweigten Alkyl-, Alkenyl-, Cycloalkyl- oder Cycloalkenyl-Rest und

$R^4$ einen gegebenenfalls substituierten, verzweigten oder unverzweigten $C_1$-$C_6$-Alkyl-Rest oder einen Cycloalkyl-Rest

bedeuten.

Bevorzugt sind Verbindungen der Formeln XVI und XVII, in denen $R_1$ für einen gegebenenfalls substituierten, verzweigten oder unverzweigten Alkyl-Rest oder Alkenyl-Rest mit bis zu 18 C-Atomen steht und in denen Acyl für Acetyl steht.

Besonders bevorzugt sind weiterhin Verbindungen der Formeln XVI und XVII, in denen $R_1$ für einen gegebenenfalls substituierten, verzweigten oder unverzweigten Alky- oder Alkenyl-Rest mit bis zu 12 C-Atomen, insbesondere bis zu 6 C-Atomen, steht und in denen Acyl für Acetyl steht.

$R_1$ und $R_4$ können außerdem für einen Cycloalkyl- oder Cycloalkenyl-Rest mit bis zu 6 C-Atomen stehen.

Das Verfahren zur Herstellung der Verbindungen mit den Formeln XVI und XVII besteht in der Umsetzung von 2-Halogenacetylacrylsäureestern der Formel III

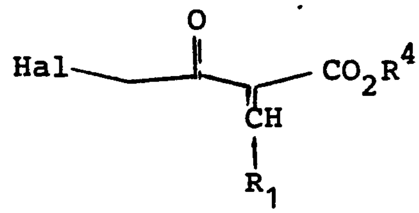

(III)

in der

$R_1$ einen gegebenenfalls substituierten verzweigten oder unverzweigten Alkyl-, Alkenyl-, Cycloalkyl- oder Cycloalkenyl-Rest und

$R_4$ einen gegebenenfalls substituierten, verzweigten oder unverzweigten $C_1$-$C_6$-Alkyl-Rest oder einen Cycloalkyl-Rest

bedeuten, mit einem Acylthioharnstoff der Formel XV, bevorzugt mit Acetylthioharnstoff.

Die Umsetzung von III mit XV kann in einem polaren Lösungsmittel wie beispielsweise Acetonitril, Aceton, Dimethylformamid oder Dimethylsulfoxid oder in einem polaren, Hydroxylgruppen-haltigen Lösungsmittel wie beispielsweise Methanol, Ethanol oder Wasser oder auch in einem Lösungsmittelgemisch wie beispielsweise Ethanol/Wasser oder Dimethylformamid/Wasser in einem Temperaturbereich zwischen 20°C und 150°C, vorzugsweise zwischen 60°C und 90°C durchgeführt werden. Die bevorzugten Produkte XVI und XVII können nach Durchführung der Reaktion und Abkühlen der Reaktionslösung durch direkte Kristallisation aus dem Reaktionsgemisch isoliert werden.

Weiterhin wurde gefunden, daß diese Reaktion durch geeignete Wahl der Reaktionsbedingungen derart geführt werden kann, daß nach Umsetzung eines Isomerengemischs III mit XV nur das bevorzugte Z-isomere Reaktionsprodukt XVI isomerenrein auskristallisiert. Dazu wird bevorzugt ein Gemisch aus Dimethylformamid und Wasser als Reaktionsmedium verwendet, das bis zu 80 %, bevorzugt 30 bis 50 %, Wasser enthält.

Weiterhin wurde gefunden, daß aus den Verbindungen der Formeln XVI und XVII durch gleichzeitige alkalische Spaltung des Amids und der Esterfunktion $COOR^4$ die Produkte VI und XVIII in einem Schritt herstellbar sind.

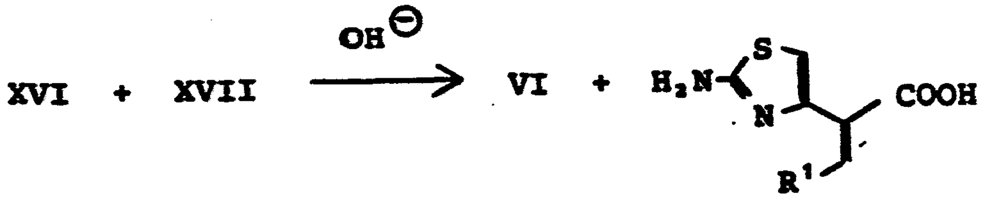


XVIII

Dabei kann aus einem Gemisch aus XVI und XVII ein Gemisch aus VI und XVIII hergestellt werden. Bei Wahl eines geeigneten Acylrestes, eines geeigneten Restes $R_4$ und geeigneter Reaktionsbedingungen ist es darüberhinaus möglich, aus der reinen Verbindung XVI stereospezifisch die reine besonders bevorzugte Verbindung VI und aus der reinen Verbindung XVII stereospezifisch die reine Verbindung XVIII zu synthetisieren. Geeignete Reste $R^4$ sind beispielsweise Methyl oder Ethyl. Als Lösungsmittel für diesen Schritt eignen sich besonders protische Lösungsmittel oder Lösungsmittelgemische wie beispielsweise Ethanol/Wasser, Methanol/Wasser oder Wasser, als alkalisches Reagens können Alkalimetallhydroxide wie Lithiumhydroxid, Natriumhydroxid oder vorteilhaft Kaliumhydroxid eingesetzt werden. Die Reaktion läuft bei Zimmertemperatur, bevorzugt bei erhöhten Temperaturen innerhalb von 1 bis 24 Stunden ab. Besonders vorteilhaft für das Verfahren

0161617

ist es, die Reaktion bei Temperaturen zwischen 60°C und 100°C durchzuführen. Weiterhin ist es vorteilhaft, das Alkalimetallhydroxid im 2 bis 10-fachen, bevorzugt in 2 bis 5-fachen molaren Überschuß einzusetzen und zwar als 1 bis 6 molare Lösung. Die Produkte VI und/ oder XVIII werden nach Durchführung der Reaktion durch Ansäuern der Reaktionslösung mit beispielsweise konzentrierter Salzsäure oder Trifluoressigsäure bei pH 3 bis 4 in hoher Reinheit ausgefällt.

Weiterhin wurde gefunden, daß es möglich ist, in den Verbindungen XVI bzw. XVII die Esterfunktion $CO_2R^4$ selektiv unter Erhalt der Amidstruktur zu den Carbonsäuren IXX bzw. XX zu spalten, in denen Acyl die obengenannte Bedeutung hat.

XVI 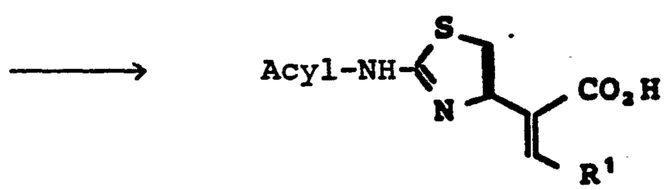

IXX

XVII 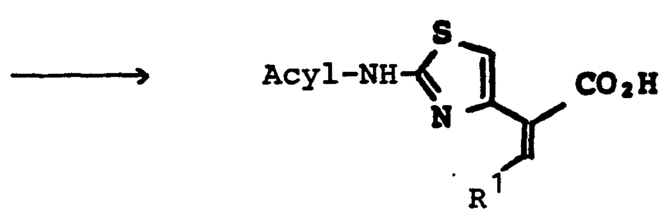

XX

Bevorzugt sind Verbindungen der Formeln IXX und XX, in denen Acyl für Acetyl steht.

Die Verbindungen IXX und XX können als Zwischenprodukte für Cephalosporin-Synthesen verwendet werden, sie können aber auch analog zu der oben beschriebenen Umsetzung XVI $\rightarrow$ VI und XVII $\rightarrow$ XVIII zu VI bzw. XVIII gespalten werden.

Als Lösungsmittel und als Basen können für die Reaktion XVI $\rightarrow$ IXX und XVII $\rightarrow$ XX die für die Reaktion XVI $\rightarrow$ VI und XVII $\rightarrow$ XVIII genannten verwendet werden. Dabei ist es vorteilhaft für das Verfahren, wenn man die Reaktion bei Raumtemperatur innerhalb von 2 bis 4 Stunden durchführt.

Beispiel 1

2-(2-Acetamidothiazol-4-yl)-2-butensäureethylester

Eine Lösung von 10,4 Gew.-Teilen Acetylthioharnstoff und 16,4 Gew.-Teilen 2-Ethyliden-4-chlor-3-oxobuttersäureethylester in 40 Vol-Teilen N,N-Dimethylformamid wird 2 Stunden auf 80°C erwärmt.

Nach dem Abkühlen wird unter Rühren in 100 Vol.-Teile Wasser eingegossen, 10 Minuten gerührt und der Niederschlag abgesaugt.

Man erhält 19,3 Gew.-Teile Produkt.

NMR ($CDCl_3$):

Z-Isomeres: $\delta$ = 10.30 (1) s breit, 6.97 (1) s, 6,81 (1) q, J=7 Hz, 4.36 (2) q, J=7 Hz, 2.17 (3) s, 1.97 (3) d, J=7 Hz, 1.34 (3) t, J=7 Hz ppm.

E-Isomeres: $\delta$ = 10.60 (1) s breit, 7.16 (1) q, J=7 Hz, 6.92 (1) s, 4.20 (2) q, J=7 Hz, 2.13 (3) s, 1.87 (3) d, J=7 Hz, 1.20 (3) t, J=7 Hz ppm.

**Beispiel 2**

**2-(2-Acetamidothiazol-4-yl)-2-butensäure**

5 Gew.-Teile 2-(2-Acetamidothiazol-4-yl)-2-butensäure-ethylester werden unter Kühlung bei 10°C in 40 Vol.-Teilen 2 n Natronlauge gelöst. Man rührt die Lösung 3,5 Stunden bei Raumtemperatur, stellt dann mit 6 n Salzsäure unter Eiskühlung auf pH 3,6, verdünnt evtl. mit etwas Wasser und saugt ab. Der Rückstand wird mit kaltem Wasser gewaschen und getrocknet, man erhält 3,6 Gew.-Teile reines Produkt.

NMR (DMSO=$d_6$):

E-Isomeres: $\delta$ = 7.01 (1) s, 6.90 (1) q, J=7 Hz, 2.10 (3) s, 1.82 (3) d, J=7 Hz ppm.

Z-Isomeres: $\delta$= 6.93 (1) s, 6.42 (1) q, J=7 Hz, 2.12 (3) s, 1.86 (3) d, J=7 Hz ppm.

**Beispiel 3**

**2-(2-Aminothiazol-4-yl)-2-butensäure**

8,4 Gew.-Teile 2-(2-Acetamidothiazol-4-yl)-2-butensäure-ethylester werden unter Rühren zu einer auf 90°C erwärmten Lösung von 18,8 Gew.-Teilen Kaliumhydroxid in 168 Vol.-Teilen Wasser gegeben. Man rührt 45 Minuten bei gleicher Temperatur, kühlt ab und stellt unter Kühlung mit konz. Salzsäure auf pH 3,6 ein.

Nach 30 Minuten wird abgesaugt, man erhält 3,6 Gew.-Teile Produkt.

Beispiel 4

Z-2-(2-Acetamidothiazol-4-yl)-2-butensäureethylester

14,6 Gew.-Teile Acetylthioharnstoff, 30 Vol.-Teile Wasser und 50 Vol-Teile N,N-Dimethylformamid werden unter Stickstoff auf 85°C aufgeheizt. Anschließend werden bei dieser Temperatur innerhalb von 40 Minuten 22,9 Gew.-Teile 2-Ethyliden-4-chlor-3-oxo-buttersäureethylester dazugegeben. Man rührt 1 Stunde bei 80 bis 85°C, kühlt dann auf 15°C ab und saugt nach 10 Minuten den ausgefallenen Niederschlag ab. Man schlämmt einmal mit 34,5 Vol.-Teilen Wasser auf, saugt erneut ab und wäscht noch zweimal mit Wasser. Das Rohprodukt wird getrocknet und dann einmal in einem Gemisch aus 13,8 Vol.-Teilen Wasser und 41,8 Vol.-Teilen Ethanol 10 Minuten ausgerührt, abgesaugt und mit Ethanol/Wasser (3/1) nachgewaschen. Nach Trocknen erhält man 8,8 Gew.-Teile reines Produkt.

Beispiel 5

2-Ethyliden-4-chlor-3-oxobuttersäureethylester

In einer Mischung von 32,9 Gew.-Teilen 4-Chloracetessigsäureethylester und 9,6 Gew.-Teilen Acetaldehyd werden bei -20°C 0,25 Gew.-Teile Piperidin eingetropft. Die

Mischung wird 5 bis 6 Stunden bei -20°C stehengelassen, dann mit eiskalter 1 n Salzsäure und Essigester versetzt. Die Essigesterphase wird abgetrennt, noch zweimal mit 1 n Salzsäure und dann einmal mit Wasser extrahiert, über Magnesiumsulfat getrocknet und eingeengt. Man erhält 35,6 Gew.-Teile eines gelben Öls, das am Kugelrohr destilliert wird (Ofentemperatur 80 bis 110°C bei 0,07 mm). Man erhält 15,5 Gew.-Teile Produkt.

Beispiel 6

Z-2-(2-Acetamidothiazol-4-yl)-2-butensäureethylester

208 Gew.-Teile Acetylthioharnstoff und 328 Gew.-Teile 2-Ethyliden-4-chlor-3-oxobuttersäureethylester werden in 800 Vol.-Teilen N,N-Dimethylformamid gelöst. Man erwärmt 2 Stunden auf 85°C, versetzt mit 2.000 Vol.-Teilen Wasser und kühlt auf 20°C ab. Der ausgefällene Niederschlag wird abgesaugt und zweimal mit je 1.200 Vol.-Teilen Ether 15 Minuten lang verrührt und erneut abgesaugt. Umkristallisation des Rückstandes aus einem Gemisch aus 100 Vol.-Teilen Ethanol und 400 Vol.-Teilen Wasser liefert 130 Gew.-Teile reinen Produkts. Aus den etherischen bzw. ethanolisch-wäßrigen Mutterlaugen läßt sich weiteres Produkt als E/Z-Gemisch gewinnen.

## Beispiel 7

### E-2-(2-Acetamidothiazol-4-yl)-2-butensäure

Zu einer Lösung von 12 Gew.-Teilen Natriumhydroxid in 130 Vol.-Teilen Wasser gibt man 22 Gew.-Teile E-2-(2-Acetamidothiazol-yl)-2-butensäureethylester. Nach 90-minütigem Rühren bei 25°C wird unter Eiskühlung mit 750 Vol.-Teilen Wasser versetzt und mit 2 n Salzsäure auf pH 2,8 eingestellt. Das ausgefallene Produkt wird abgesaugt, zweimal mit je 50 Vol.-Teilen Wasser gewaschen und getrocknet. Man erhält 17,9 Gew.-Teile der Titelverbindung.

## Beispiel 8

### 2-Ethyliden-4-chlor-3-oxobuttersäureethylester

50 Gew.-Teile 4-Chloracetessigsäureethylester und 76 Vol.-Teile Ethylacetat werden vorgelegt, mit Stickstoff überlagert und auf -75°C abgekühlt. Zur kalten Lösung gibt man 33 Gew.-Teile Acetaldehyd und läßt anschließend eine Lösung von 0,43 Vol.-Teilen Piperidin in 3 Vol.-Teilen Ethylacetat innerhalb von 50 Minuten zufließen. Nun wird die Lösung innerhalb von 40 Minuten auf 0°C erwärmt und 2 Stunden bei dieser Temperatur gerührt. Aufarbeitung erfolgt nach Zugabe von 121 Vol.-Teilen 1 n Salzsäure und 16 Gew.-Teilen Kochsalz. Man trennt die organische Phase ab und extrahiert die wäßrige Phase nochmals mit 45 Vol.-Teilen Ethylacetat.

Die vereinigten organischen Phasen werden zweimal mit einer Lösung von je 16 Gew.-Teilen Kochsalz in 55 Vol.-Teilen Wasser extrahiert und getrocknet. Nach Abziehen des Lösungsmittels verbleiben 51,9 Gew.-Teile Produkt, welches roh weiterverarbeitet werden kann.

Beispiel 9

Z-2-(2-Aminothiazol-4-yl)-2-butensäure

Eine Lösung von 16,5 Gew.-Teilen Kaliumhydroxid in 111 Vol.-Teilen Wasser wird auf 75 bis 78°C aufgeheizt. Man gibt 14,9 Gew.-Teile Z-2-(2-Acetamidothiazol-4-yl)-2-butensäureethylester zu und rührt 2 Stunden bei 75 bis 78°C. Nach Abkühlen auf 10°C wird mit Trifluoressigsäure pH 7,0 eingestellt, die Lösung über Kieselgur abgesaugt und anschließend mit weiterer Trifluoressigsäure pH 3,6 eingestellt. Man kühlt auf 0°C, rührt 30 Minuten bei 0°C und isoliert die ausgefallenen Kristalle. Nach dreimaligem Waschen mit je 6 Vol.-Teilen Isopropanol wird getrocknet. Das Produkt wird dann 20 Minuten mit 62,4 Vol.-Teilen Aceton verrührt, abgesaugt, mit 42 Vol.-Teilen Aceton gewaschen und erneut getrocknet. Man erhält 6,3 Gew.-Teile reines Produkt. Aus der Mutterlauge der Aceton-Ausrührung kann weiteres Produkt isoliert werden.

## Beispiel 10

### Z-2-(2-Acetamidothiazol-4-yl)-2-butensäureethylester

In einem Dreihalskolben mit Rührer, Rückflußkühler, Thermometer und Tropfrichter werden 150 Gew.-Teile 4-Chloracetessigsäureethylester in 250 Gew.-Teilen Dichlormethan gelöst und auf 10°C abgekühlt. Hierzu werden 100 Gew.-Teile Acetaldehyd auf einmal zugegeben. Bei 10 °C unter Rühren wird dann eine Lösung aus 1,38 Vol.-Teilen Piperidin in 25 Vol.-Teilen Dichlormethan so zugetropft, daß die Innentemperatur zwischen 10 und 20°C gehalten wird. Nach beendetem Zutropfen wird bei 20°C 1,5 Stunden nachgerührt. Anschließend gibt man 107,6 Gew.-Teile Acetylthioharnstoff, 225 Vol.-Teile Dimethylformamid und 125 Vol.-Teile Wasser zum Ansatz und schließt eine Destillationsbrücke an. Man heizt mit einem 80°C warmen Bad auf. Wenn nichts mehr überdestilliert und die Innentemperatur die 80°C-Marke erreicht hat, läßt man bei 80°C nachrühren und kühlt anschließend auf 10 bis 15°C ab. Es wird 30 Minuten bei 10 bis 15°C gerührt, abgesaugt, mit der Mutterlauge nachgewaschen und trockengesaugt. Das trockengesaugte Produkt wird dreimal mit je 90 Vol.-Teilen Wasser nachgewaschen und getrocknet. Das Rohprodukt wird mit 241 ml Ethanol/Wasser (3:1) 30 Minuten verrührt, abgesaugt, mit Ethanol/Wasser (3:1) gewaschen und getrocknet. Man erhält 52,3 Gew.-Teile reines Produkt.

Patentansprüche

1. Verbindungen der Formeln XVI und XVII

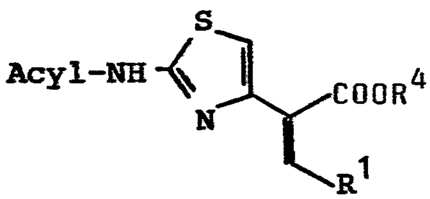

XVI

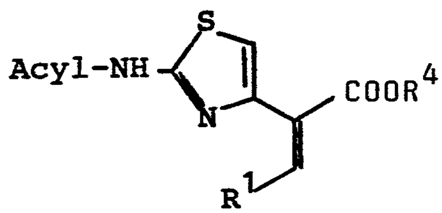

XVII

in denen

Acyl Alkylcarbonyl, bevorzugt $C_1$-$C_6$-Alkylcarbonyl, worin Alkyl verzweigt oder unverzweigt und auch cyclisch sein kann und durch Halogen oder Aryl substituiert sein kann, wenn durch Aryl, dann bevorzugt Phenyl, Arylcarbonyl, bevorzugt Phenylcarbonyl oder durch Niedrigalkoxy, Halogen oder Nitro substituiertes Phenylcarbonyl,

$R^1$ einen gegebenenfalls substituierten verzweigten oder unverzweigten Alkyl-, Alkenyl-, Cycloalkyl- oder Cycloalkenyl-Rest und

$R^4$ einen gegebenenfalls substituierten, verzweigten oder unverzweigten $C_1$-$C_6$-Alkyl-Rest oder einen Cycloalkyl-Rest bedeuten.

2. Verbindungen nach Anspruch 1, dadurch gekennzeichnet, daß $R_1$ für einen gegebenenfalls substituierten, verzweigten oder unverzweigten Alkyl- oder Alkenyl-Rest mit bis zu 18 C-Atomen und Acyl für Acetyl steht.

3. Verbindungen nach Anspruch 1, dadurch gekennzeichnet, daß $R_1$ für einen gegebenenfalls substituierten, verzweigten oder unverzweigten Alkyl- oder Alkenyl-Rest mit bis zu 12 C-Atomen, insbesondere bis zu 6 C-Atomen und Acyl für Acetyl steht.

4. Verbindungen nach Anspruch 1, dadurch gekennzeichnet, daß $R_1$ und $R_4$ einen Cycloalkyl- oder Cycloalkenyl-Rest mit bis zu 6 C-Atomen bedeuten.

5. Verfahren zur Herstellung von Verbindungen der Formeln XVI und XVII

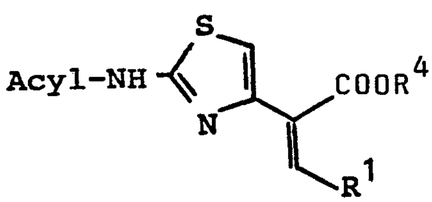

XVI

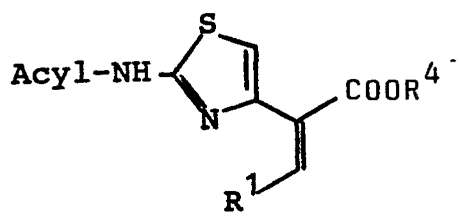

XVII

dadurch gekennzeichnet, daß man einen 2-Halogenacetylacrylsäureester der Formel III

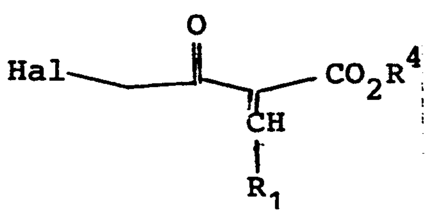

(III)

in der

$R_1$ einen gegebenenfalls substituierten verzweigten oder unverzweigten Alkyl-, Alkenyl-, Cycloalkyl- oder Cycloalkenyl-Rest und

$R_4$ einen gegebenenfalls substituierten, verzweigten oder unverzweigten $C_1$-$C_6$-Alkyl-Rest oder einen Cycloalkyl-Rest bedeuten,

mit einem Acylthioharnstoff der Formel XV

$$\text{Acyl-HN-}\overset{\overset{\displaystyle S}{\|}}{C}\text{-NH}_2 \qquad \text{(XV)}$$

worin Acyl für Alkylcarbonyl, bevorzugt $C_1$-$C_6$-Alkylcarbonyl, worin Alkyl verzweigt und unverzweigt und auch cyclisch sein kann und durch Halogen oder Aryl substituiert sein kann, wenn durch Aryl, dann bevorzugt Phenyl, Arylcarbonyl, bevorzugt Phenylcarbonyl oder durch Niedrigalkoxy, Halogen oder Nitro substituiertes Phenylcarbonyl steht, umsetzt.

6. Verfahren nach Anspruch 5, dadurch gekennzeichnet, daß man die Umsetzung in Acetonitril, Aceton, Dimethylformamid, Dimethylsulfoxid, Methanol, Ethanol oder Wasser oder in einem Dimethylformamid/Wasser oder Ethanol/Wasser-Gemisch bei Temperaturen von 20 bis 150°C durchführt und die Reaktionsprodukte durch direkte Kristallisation aus dem Reaktionsgemisch isoliert.

7. Verfahren zur Herstellung der Verbindungen der Formel XVI nach Anspruch 5, dadurch gekennzeichnet, daß man als Reaktionsmedium ein Dimethylformamid/Wasser-Gemisch verwendet, das bis zu 80 % Wasser enthält und das Z-Isomere aus dem Reaktionsgemisch durch Umkristallisation isomerenrein isoliert.

8. Verwendung von Verbindungen der Formeln XVI und XVII nach Anspruch 1 zur Herstellung von Verbindungen der Formeln VI und XVIII

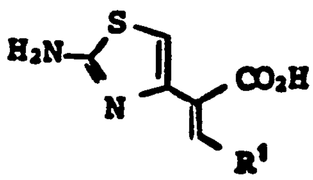

VI

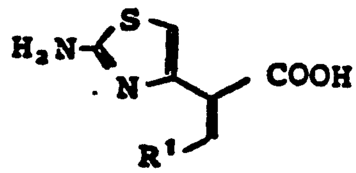

XVIII

in denen $R^1$ die in Anspruch 1 angegebene Bedeutung hat,

durch alkalische Spaltung der Acylamid- und Esterfunktionen.

9. Verwendung von Verbindungen der Formeln XVI und XVII nach Anspruch 1, zur Herstellung von Verbindungen der Formeln IXX und XX

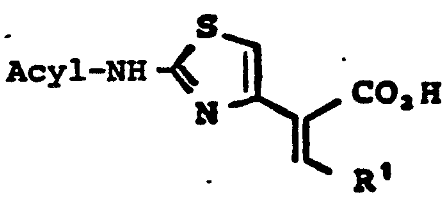

IXX

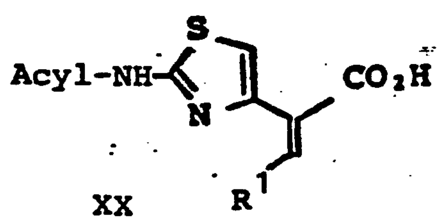

XX

in denen Acyl und $R^1$ die in Anspruch 1 angegebene Bedeutung haben,

durch selektive alkalische Spaltung der Esterfunktionen.

10. Verwendung von Verbindungen der Formeln IXX und XX

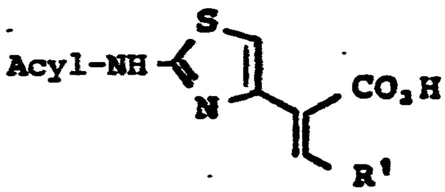

IXX

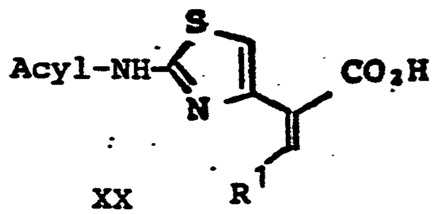

XX

in denen Acyl und $R^1$ die in Anspruch 1 angegebene Bedeutung haben,

zur Herstellung von Cephalosporinen.